# FASCICULE DE BREVET EUROPEEN

(11) **EP 1 303 237 B1**
(45) Date de publication et mention de la délivrance du brevet: **13.10.2004**
(21) Numéro de dépôt: 01934097.5
(22) Date de dépôt: 11.05.2001
(51) Int. Cl.: A61F 5/00

(54) **ANNEAU DE GASTROPLASTIE REGLABLE**
EINSTELLBARES MAGENBAND
ADJUSTABLE GASTROPLASTY RING COMPRISING A GRIP TAB

(30) Priorité: 12.05.2000 FR 0006329
(43) Date de publication de la demande: 23.04.2003
(73) Titulaire: Compagnie Européenne d'Etude et de Recherche de Dispositifs pour l'Implantation par Laparoscopie, 38200 Vienne (FR)
(72) Inventeur: BENCHETRIT, Salomon, F-69300 Caluire (FR)
(74) Mandataire: Martin, Didier Roland Valéry
(86) Numéro de dépôt international: PCT/FR2001/001434
(87) Numéro de publication internationale: WO 2001/085071

(56) Documents cités:
- DE-A- 19 751 733
- DE-U- 9 014 048
- US-A- 5 074 868

## Description

La présente invention se rapporte au domaine technique des implants chirurgicaux destinés à traiter l'obésité par implantation d'une bande gastrique souple destinée à resserrer l'estomac d'un patient, ladite bande gastrique étant pourvue d'une chambre de compression annulaire à volume variable et réglable par l'intermédiaire d'un cathéter de réglage relié à un dispositif de réglage et de commande implanté dans le corps du patient.

La présente invention concerne un anneau de gastroplastie formé par une bande souple qui comprend une première et une deuxième parties d'extrémité et qui est destinée à être fermée autour de l'estomac sensiblement vers et par ses deux extrémités, à l'aide d'un système de fermeture pour réduire le diamètre de l'ouverture de stoma en formant une boucle, la bande comportant une chambre de compression annulaire à volume réglable reliée, au niveau de l'une des parties d'extrémité, par un cathéter de réglage à un dispositif de réglage de la pression interne de ladite chambre, de manière à régler son expansion diamétrale.

### TECHNIQUE ANTERIEURE

Dans le cas de patients atteints d'obésité extrêmement sévère (obésité morbide), c'est-à-dire dans le cas de patients dont le poids excède le poids idéal d'au moins cinquante kilos par exemple, il est absolument nécessaire d'intervenir de manière chirurgicale sur de tels patients afin d'éviter non seulement une série de problèmes de santé découlant de cette obésité, mais encore pour éviter une mort certaine et proche de tels patients.

En effet, il est acquis que les patients souffrant d'obésité morbide voient leur espérance de vie réduite de manière importante et d'au moins une dizaine à une quinzaine d'années, tout en créant d'importants problèmes de charges psychologiques. Par ailleurs, toute une série de phénomènes annexes de santé sont impliqués, ayant une incidence sur l'apparition de maladies cardio-vasculaires, d'hypertension, de diabète, d'arthrites sévères notamment.

Il est également apparu que des traitements basés sur une diète sévère combinée avec une série d'exercices physiques associés à une modification du comportement, notamment alimentaire, étaient peu adaptés à de tels cas d'obésité morbide, bien que de telles méthodes de traitement soient les plus saines.

C'est la raison pour laquelle les traitements efficaces et à long terme de traitement de l'obésité morbide font intervenir un traitement chirurgical.

De manière générale, on distingue les techniques de traitement chirurgical faisant intervenir un défaut d'absorption des aliments, c'est-à-dire un raccourcissement du passage classique de l'aliment et des sucs digestifs et les techniques faisant intervenir une restriction gastrique, réduisant la taille de l'estomac.

Les techniques chirurgicales impliquant un défaut d'absorption sont par exemple celles impliquant une technique de by-pass ou dérivation du petit intestin ou encore celles mettant en oeuvre une séparation du passage des aliments relativement aux sucs digestifs. La technique chirurgicale de by-pass peut donner lieu à de sévères complications, de telle sorte que cette technique n'est plus que très rarement utilisée. La technique chirurgicale de séparation de passage du bol alimentaire relativement aux sucs digestifs ne fait pas intervenir de complications particulières, mais nécessite une intervention chirurgicale importante impliquant notamment une gastrectomie partielle.

C'est la raison pour laquelle on tend désormais à utiliser les techniques chirurgicales mettant en oeuvre une restriction gastrique pour réduire la prise d'aliments.

De telles techniques font intervenir de manière dassique l'utilisation d'anneaux de gastroplastie implantés autour de l'estomac pour réduire sa taille et le diamètre de son passage (stoma).

La plupart des dispositifs de gastroplastie connus, et par exemple celui décrit dans le brevet US-A-5074868, mettent en oeuvre une bande souple réalisée en matériau élastomère et destinée à être implantée autour de l'estomac puis resserrée et fermée suivant une boucle de diamètre fixe par un système de fermeture. Le corps de la bande souple comporte une cavité ou chambre de compression à volume variable reliée par un cathéter de réglage à un dispositif de réglage de la pression interne de la chambre afin de faire varier le diamètre interne de la boucle pour modifier ou régler le diamètre du stoma par injection ou extraction d'un volume de liquide hors de la chambre. Une telle opération de réglage du diamètre interne de l'anneau s'effectue à l'aide de dispositifs dassiques de commande incluant un boîtier miniaturisé implanté directement sous la peau du patient et pourvu d'une membrane auto-obturante à travers laquelle le médecin injecte ou retire du liquide par une seringue.

Le système de fermeture du brevet US-A-5074868 met en oeuvre un suturage, à l'aide de fils de suture, des deux brins de la bande souple de l'anneau.

Un tel dispositif donne généralement satisfaction, mais souffre comme la plupart des systèmes connus, d'inconvénients liés essentiellement à la difficulté de toute intervention chirurgicale susceptible de survenir après la pose de l'implant de gastroplastie. En effet, il s'avère que malgré la possibilité de modifier dans une certaine mesure le diamètre de l'anneau sans intervention chirurgicale par le boîtier miniaturisé mentionné ci-dessus, la pose de tels implants gastriques peut s'accompagner de phénomènes d'intolérance, par exemple accompagné de vomissements, liés à une trop forte réduction du diamètre de stoma, ou encore à une action inefficace de l'implant lié à un diamètre du stoma trop important, ou encore tout simplement à une gêne ou une infection ou inflammation locale ou générale.

C'est la raison pour laquelle il est souvent nécessaire d'intervenir à nouveau de manière chirurgicale, soit pour soulager le patient, soit pour modifier ou changer l'anneau de gastroplastie préalablement implanté. De telles interventions chirurgicales sont particulièrement sévères et nécessitent de plus, soit la découpe de l'anneau par un chirurgien, soit la coupe du fil de suture s'accompagnant d'une ouverture complète de l'anneau puis son changement et son remplacement.

De telles opérations sont délicates à réaliser, sont difficilement supportées par le patient et sont donc coûteuses dans la mesure où elles impliquent la destruction d'un implant et son remplacement.

### EXPOSE DE L'INVENTION

L'objet assigné à l'invention vise en conséquence à proposer un nouvel anneau de gastroplastie permettant de porter remède aux différents inconvénients énumérés précédemment et susceptible de faciliter la manipulation de l'anneau lors de la pose de l'implant ainsi que lors de la réouverture et la re-fermeture de l'anneau au cours d'une nouvelle opération.

Un autre objet de l'invention vise à proposer un nouvel anneau de gastroplastie susceptible d'assurer de manière simple et fiable une fermeture réversible de la boucle constituant l'anneau tout en permettant un déverrouillage aisé des parties d'extrémité et ce, sans entraîner la destruction de l'implant.

Un autre objet de l'invention vise à proposer un nouvel anneau de gastroplastie susceptible de proposer des moyens simples et fiables d'adaptation du diamètre de l'anneau à chaque situation chirurgicale donnée.

Un autre objet de l'invention vise à proposer un nouvel anneau de gastroplastie susceptible de présenter plusieurs diamètres d'implantation.

Un autre objet de l'invention vise à proposer un nouvel anneau de gastroplastie permettant de réduire la gêne ressentie par le patient tout en étant fermement maintenu en place par la boucle.

Un autre objet de l'invention vise à proposer un nouvel anneau de gastroplastie particulièrement facile à fabriquer tout en étant d'une excellente résistance mécanique générale.

Les objets assignés à l'invention sont atteints à l'aide d'un anneau de gastroplastie formé par une bande souple qui comprend une première et une deuxième parties d'extrémité et qui est destinée à être fermée autour de l'estomac vers ses deux parties d'extrémité par un système de fermeture pour réduire le diamètre de l'ouverture du stoma en formant une boucle, caractérisé en ce que l'anneau comprend des pattes de préhension qui font saillie vers l'extérieur de la boucle pour faciliter le rapprochement et l'écartement des deux parties d'extrémité.

### DESCRIPTIF SOMMAIRE DES DESSINS

D'autres objets et avantages de l'invention apparaîtront mieux à la lecture de la description ci-jointe, ainsi qu'à l'aide des dessins annexés, à titre purement illustratif et informatif dans lesquels :
- La figure 1 illustre selon une vue schématique en perspective, un exemple d'un mode de réalisation d'un anneau de gastroplastie conforme à l'invention en position d'ouverture avant son implantation.
- La figure 2 illustre selon une vue schématique en perspective, un exemple d'un mode de réalisation d'un anneau de gastroplastie conforme à l'invention en position de fermeture.
- La figure 3 illustre selon une vue de dessus l'anneau de gastroplastie de la figure 2.

### MEILLEURE MANIERE DE REALISER L'INVENTION

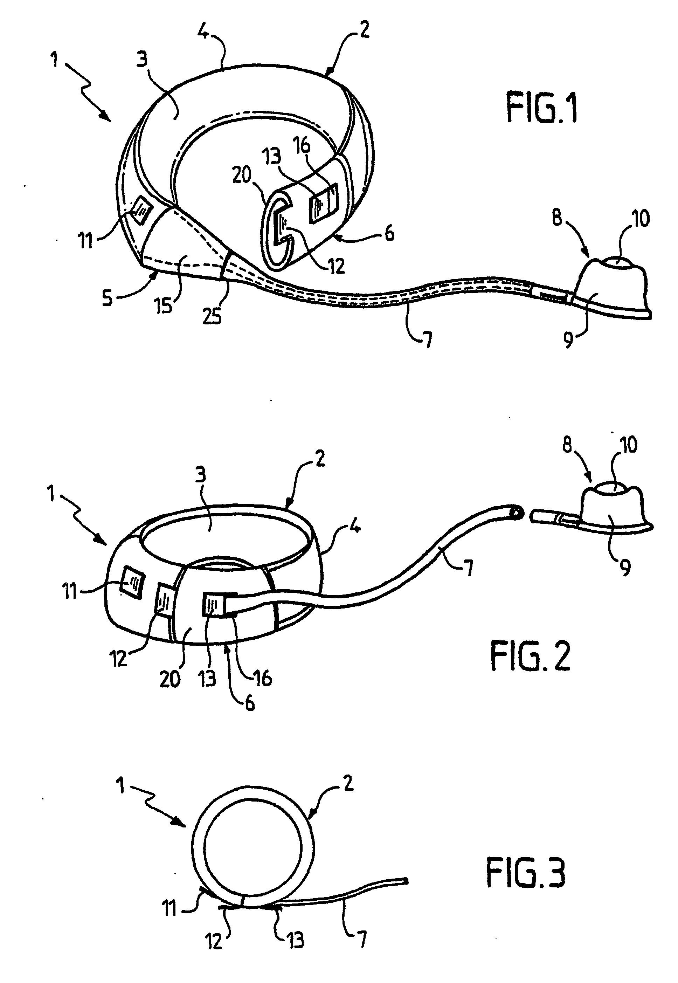

Les figures 1 et 2 illustrent un exemple de réalisation préférentielle d'un anneau de gastroplastie 1 conforme à l'invention, formé par une bande souple 2 réalisée par exemple par thermoformage à partir d'un matériau élastomère à usage chirurgical, ladite bande 2 définissant, de préférence sensiblement sur toute sa longueur, une chambre de compression interne 3, délimitée par les parois 4 de la bande souple 2 et par une première et une deuxième parties d'extrémité 5, 6.

Dans sa position d'implantation dans l'estomac d'un patient, telle qu'illustrée à la figure 2, la chambre de compression 3 forme donc une chambre de compression annulaire.

Tel que cela est bien connu dans l'art antérieur, la chambre de compression 3 définit un volume fermé et interne à l'anneau de gastroplastie, qui est destiné à former un volume réglable de manière à régler l'expansion diamétrale de l'anneau lorsqu'il est en place pour l'adapter à chaque situation chirurgicale donnée.

De manière classique, le réglage de l'expansion diamétrale de l'anneau de gastroplastie conforme à l'invention est assuré par un cathéter de réglage 7 formé par un élément tubulaire en matériau élastomère prolongeant l'une des parties d'extrémité libre, par exemple la première partie d'extrémité 5, de la chambre de compression 3 pour relier ladite chambre à un dispositif de réglage 8 de la pression interne de ladite chambre.

Tel que cela est bien connu de l'homme de l'art, le dispositif de réglage 8 peut être formé par un boîtier miniaturisé 9 implanté sous la peau du patient. Le boîtier miniaturisé 9 comporte par exemple une membrane supérieure 10 auto-obturante destinée à être percée par une seringue pour injecter ou retirer une certaine quantité de fluide servant à assurer la variation de volume de la chambre de compression 3, pour régler le volume de la chambre et obtenir ainsi le diamètre interne de l'anneau souhaité. Un tel dispositif étant bien connu de l'homme du métier, il ne sera en conséquence pas décrit plus en détail.

L'anneau de gastroplastie conforme à l'invention comporte également un système de fermeture destiné à fermer et maintenir en position de boude l'anneau de gastroplastie autour de l'estomac.

Selon une caractéristique importante de l'invention, l'anneau comprend en outre au moins une patte de préhension qui fait saillie vers l'extérieur de la boucle pour faciliter le rapprochement et l'écartement des deux parties d'extrémité lors de la fermeture de l'anneau et/ou lors d'une ré-opération afin d'ajuster l'anneau, de le déverrouiller ou bien encore de le re-verrouiller.

Comme cela est représenté aux figures, la bande souple 2 possède, au voisinage de la première partie d'extrémité 5 de l'anneau, au moins une première patte de préhension 11.

De préférence, la deuxième partie d'extrémité 6 comprend une deuxième patte de préhension 12 et, avantageusement, elle porte également une troisième patte de préhension 13.

Chacune des pattes de préhension 11, 12, 13 est, de préférence, sensiblement plane et s'étend entre une première extrémité qui est solidaire des parois de l'anneau et une deuxième extrémité qui est libre et qui peut être saisie par pincement pour déplacer positivement au moins l'une des parties d'extrémité 5 et 6.

De préférence encore, les pattes de préhension sont souples pour pouvoir s'effacer lors de l'introduction de l'anneau dans le corps du patient. Elles sont de forme rectangulaire.

Chacune des pattes de préhension s'étend tangentiellement aux parois 4 de l'anneau 1 de sorte qu'elle est perpendiculaire au plan de cet anneau et est de faibles dimensions par rapport aux dimensions générales de l'anneau. En particulier, l'épaisseur des pattes est fine par rapport à leur largeur et leur longueur.

Les pattes de préhension sont venues de matière avec la bande et les parois des parties d'extrémité 5 et 6 ou sont rapportées et solidarisées avec celles-ci par tout moyen mécanique ou chimique, comme par exemple par collage ou surmoulage, en étant réalisées en un matériau élastomère biocompatible.

De manière générale, les pattes de préhension forment des excroissances qui peuvent être implantées en tout point de la surface externe ou de la surface interne des parois de la bande et des parties d'extrémité, pour être saisies par le chirurgien à l'aide de tout instrument chirurgical tel que par exemple une pince, afin de faciliter la manipulation de l'anneau, son verrouillage et son déverrouillage.

De préférence encore, la première patte de préhension 11 s'étend en direction opposée à la deuxième partie d'extrémité 6. La deuxième patte de préhension 12 s'étend dans la même direction que la première patte 11 tandis que la troisième patte 13 est en direction opposée aux deux autres pattes 11 et 12.

Selon une caractéristique de l'invention, le système de fermeture conforme à l'invention comporte des moyens de blocage et desserrage de l'anneau qui sont portés par le cathéter de réglage 7, permettant d'assurer, à partir de la position de blocage diamétral de l'anneau correspondant à la position fermée et en boucle telle qu'illustrée à la figure 2, son relâchement diamétral momentané par déplacement relatif des deux parties d'extrémité 6, 7 de l'anneau, tout en formant si on le souhaite, une boucle fermée autour de l'estomac. Avantageusement, les moyens de blocage et desserrage sont réversibles.

Selon l'invention, les moyens de blocage et de desserrage comportent des moyens pneumatiques pour assurer la fermeture et l'ouverture de l'anneau, faisant intervenir un fluide, un gaz ou un liquide par exemple. Le recours à des moyens pneumatiques permet une mise en place simplifiée de l'implant et une commande simple de son ouverture et fermeture.

La réalisation d'une telle fonction technique permet, en plus de la simplicité de mise en place et fermeture de l'anneau qu'elle procure, de réduire la gravité et l'importance d'éventuelles ré-interventions chirurgicales suivant la pose d'un implant en évitant d'avoir à sectionner et détruire l'anneau de gastroplastie mis en place. Une telle fonction permet d'ouvrir facilement l'anneau à l'aide des première et troisième pattes de préhension 11,13 et même de la deuxième patte, sans détruire la boude de l'anneau, de laisser éventuellement l'anneau en place et de permettre un re-verrouillage ultérieur à l'aide de la deuxième patte 12.

Selon une variante préférentielle de l'invention, telle qu'illustrée aux figures 1 et 2, l'anneau de gastroplastie conforme à l'invention comprend un système de fermeture dont les moyens de blocage et desserrage réversibles comportent au moins une zone déformable 15 et une ouverture 16 ménagée dans la paroi 4 de la deuxième partie d'extrémité 6 de la bande 2. Le cathéter de réglage 7 gonflable qui est réalisé en un matériau élastomère biocompatible, est destiné à être enfilé dans l'ouverture 16 dans la position de fermeture de l'anneau et à servir en outre de moyen de guidage.

La zone déformable 15 est susceptible de former une excroissance en cas d'augmentation de pression dans le cathéter de réglage 7, ladite excroissance prenant appui contre les parois 4 de la partie d'extrémité 6 de la bande, à l'intérieur de la chambre 3 pour bloquer l'anneau en position de fermeture. L'excroissance reprend sa forme de repos en cas de retour à la pression normale dans le cathéter de réglage 7 pour permettre le coulissement et le guidage libre dudit cathéter dans l'ouverture 16 et le desserrage de la boucle.

Selon une version particulièrement avantageuse de l'invention, la zone déformable réversible 15 est formée par au moins une zone de moindre résistance et dans sa forme de repos, constitue une zone de forme convergente en direction de la deuxième partie d'extrémité 6. Elle peut être par exemple de forme triangulaire en section transversale c'est-à-dire, selon un plan de coupe perpendiculaire au plan de l'anneau.

Avantageusement, la zone déformable 15 peut être formée par une section du cathéter de réglage 7 présentant une dureté du matériau élastomère composant le cathéter, qui est localement inférieure à la dureté générale dudit cathéter. Dans un tel cas, le cathéter de réglage 7 étant relié au dispositif de réglage 8 externe de mise en pression par l'intermédiaire d'un fluide (air ou liquide), la zone 15 tendra à former un ballonnet d'un diamètre supérieur aux dimensions intérieures de la bande, ce qui assure le blocage diamétral de l'anneau. La mise en pression du ballonnet intervient avant celle de la bande, ce qui est facilité par la forme convergente du ballonnet.

Avantageusement, la bande souple 2 est pourvue à une partie d'extrémité, et par exemple à la deuxième partie d'extrémité 6 opposée à la première partie d'extrémité 5 qui est prolongée par le cathéter de réglage 7, d'un manchon creux 20 prolongeant la bande souple 2.

Le manchon creux 20 comporte également l'ouverture 16 qui est ménagée dans l'une de ses faces, de préférence une face externe, de telle manière que l'autre extrémité 5 de la bande souple 2 puisse s'insérer dans ledit manchon en position de fermeture, le cathéter de réglage 7 passant alors dans l'ouverture 16 (figure 2) pour former la boucle de l'anneau. Une telle disposition constructive assure une bonne fiabilité de la tenue de la fermeture, tout en permettant à la chambre de compression 2 de s'étendre sur tout le périmètre de serrage de l'estomac.

De préférence, la bande 2 et le manchon creux 20 sont de section transversale de forme oblongue pour faciliter encore le coincement de la zone déformable 15 lors de sa mise en pression.

Avantageusement, la bande souple 2, la chambre de compression 3 ainsi que le manchon creux 20 forment une pièce monobloc réalisée à partir d'un même matériau plastique élastomère, le cathéter de réglage 7 étant ensuite thermosoudé.

De préférence, la troisième patte de préhension 13 s'étend à partir de la surface externe du manchon, et en partie au-dessus de l'ouverture 16 sans toutefois gêner le passage du cathéter au travers de cette ouverture.

De manière avantageuse, il est encore possible de réaliser la zone déformable 15 avec une épaisseur différente de l'épaisseur de la bande souple 2 afin d'obtenir des débits de fluide différents dans chacun de ces éléments. Ces débits différents peuvent également être obtenus par des formes différentes et ce, afin par exemple de dégonfler la bande souple 2 avant la zone déformable 15.

La partie d'extrémité 5 de la bande 2 à laquelle est relié le cathéter de réglage 7, est de préférence de forme conique pour faciliter son insertion dans le manchon creux 20. La zone déformable 15 est située à l'intérieur de cette forme conique.

De préférence encore, le cathéter de réglage 7 possède une butée 25 qui est située à l'extrémité de la partie conique, à proximité de la zone déformable 15 et qui est destinée à traverser l'ouverture 16 du manchon creux 20. Après encliquetage de cette butée dans l'ouverture 16, l'excroissance 15 peut être bloquée par gonflage.

Ces moyens de butée 25 consistent par exemple en un surplus de matière formant un bossage tourné vers l'extérieur de l'anneau et qui évite un déverrouillage intempestif de l'anneau. L'encliquetage de ces moyens permet également de vérifier que le cathéter a été suffisamment enfilé dans l'ouverture 16 pour atteindre le diamètre nominal de l'anneau et en outre permettent d'assurer un maintien en position de l'excroissance bloquée dans le manchon creux 20.

En outre, le cathéter de réglage 7 est sensiblement rigide et est d'une longueur importante par rapport au diamètre de l'anneau pour faciliter l'opération d'enfilage et le passage du cathéter 7 dans l'ouverture 16. Ceci permet également une bonne mise en place de la bande dans la position désirée.

La réalisation d'un anneau de gastroplastie monobloc permet de simplifier le procédé de fabrication de l'anneau et d'obtenir un anneau ne présentant pas de risque d'altération dans le temps.

Lors de l'implantation, l'anneau conforme à l'invention est mis en place autour de l'estomac dans la position illustrée à la figure 1. Le dispositif de réglage 8 étant au préalable déconnecté, le chirurgien assure l'enfilage et le passage du cathéter de réglage 7 dans l'ouverture 16 pour insérer la première partie d'extrémité 5 dans le manchon creux 20 (figure 1). Le chirurgien amène ensuite la butée 25 en position de blocage dans l'ouverture 16 à l'aide de la deuxième patte de préhension 12. Il peut ensuite, par l'intermédiaire du dispositif de réglage et de gonflage 8 raccordé à l'unique cathéter 7, assurer le blocage en position de l'anneau. Le chirurgien règle ensuite le diamètre interne de l'anneau en injectant ou retirant la quantité de liquide idoine au travers du cathéter 7. On notera que l'ensemble de ces opérations est réalisé au moyen d'un seul et unique cathéter 7.

En cas de ré-opération chirurgicale, il est possible grâce à l'anneau de gastroplastie conforme à l'invention, de se limiter à réaliser, par coelioscopie ou laparoscopie, un examen superficiel extérieur de la situation de l'implant, par simple inspection optique à l'aide d'une caméra. Eventuellement, si la situation le nécessite, il est possible dans un premier temps, par simple coelioscopie de déverrouiller le cathéter. A cet effet, il suffit de mettre en dépression le cathéter 7, ce qui conduit à un dégonflage de la bande et à un relâchement du ballonnet. Il suffit ensuite de désolidariser les deux parties d'extrémité 5 et 6 par action sur les première et troisième pattes de verrouillage 11, 13. Les pattes permettent donc de dégager la butée 25 hors de l'ouverture 16. Le coulissement du cathéter à travers l'ouverture 16 peut alors être obtenu. On peut également utiliser la deuxième patte de préhension 12 pour extraire la butée et ouvrir l'anneau ; celle-ci est alors retournée sur elle-même en direction opposée à la première patte de préhension 11.

Un tel coulissement s'accompagne d'un desserrage partiel et momentané de l'anneau, sans avoir à opérer le patient de manière sévère.

On peut remarquer que l'anneau de gastroplastie selon l'invention possède des pattes de préhension qui, lors du déverrouillage, sont manipulées dans des directions opposées de sorte que l'anneau subit des efforts sensiblement symétriquement opposés. Lors du déverrouillage, l'anneau n'a ainsi pas tendance à déplacer la partie d'estomac autour de laquelle il est placé.

Il est ensuite possible, également par simple examen et intervention laparoscopique, de refermer et bloquer à nouveau l'anneau en position de fermeture de manière très simple, puisque la boucle de l'anneau n'a jamais été détruite.

On comprend ainsi que les pattes de préhension 11 à 13 ont pour fonction de faciliter le verrouillage de l'anneau et de rendre possible le déverrouillage de l'anneau sans destruction de cet anneau.

### POSSIBILITE D'APPLICATION INDUSTRIELLE

L'invention trouve son application industrielle dans la réalisation et l'utilisation d'anneaux de gastroplastie.

## Revendications

1. Anneau de gastroplastie formé par une bande souple (2) qui comprend une première (5) et une deuxième (6) parties d'extrémité et qui est destinée à être fermée autour de l'estomac vers ses deux parties d'extrémité (5, 6) par un système de fermeture pour réduire le diamètre de l'ouverture du stoma en formant une boucle, **caractérisé en ce que** l'anneau comprend au moins une patte de préhension (11, 12, 13) qui fait saillie vers l'extérieur de la boucle pour faciliter le rapprochement et l'écartement des deux parties d'extrémité (5, 6).

2. Anneau selon la revendication 1, **caractérisé en ce que** l'anneau comporte, au voisinage de la première partie d'extrémité (5), au moins une première patte de préhension (11) qui permet d'écarter la première partie d'extrémité (5) de la deuxième partie d'extrémité (6).

3. Anneau selon la revendication 2, **caractérisé en ce que** la deuxième partie d'extrémité comporte au moins une deuxième patte de préhension (12) qui permet le rapprochement des parties d'extrémité et/ou l'écartement de ces parties d'extrémité.

4. Anneau selon la revendication 3, **caractérisé en ce que** la deuxième partie d'extrémité (6) comprend en outre au moins une troisième patte de préhension (13) qui permet d'écarter la deuxième partie d'extrémité (6) de la première partie d'extrémité (5).

5. Anneau selon la revendication 4, **caractérisé en ce que** les pattes de préhension (11, 12, 13) sont de forme sensiblement plane et s'étendent entre une première extrémité qui est solidaire de l'anneau et une deuxième extrémité qui est libre et qui peut être saisie par pincement pour déplacer positivement la partie d'extrémité de l'anneau sur laquelle elle est montée.

6. Anneau selon la revendication 5, **caractérisé en ce que** la première patte de préhension (11) est dirigée en direction opposée à la deuxième partie d'extrémité (6), **en ce que** la deuxième patte de préhension (12) est orientée dans la même direction que la première patte de préhension (11) et **en ce que** la troisième patte de préhension (13) est opposée à la deuxième patte de préhension (12).

7. Anneau selon l'une des revendications précédentes **caractérisé en ce que** la bande (2) comporte une chambre de compression annulaire (3) à volume réglable reliée, au niveau de la première partie d'extrémité (5), par un cathéter de réglage (7) à un dispositif de réglage de la pression interne de ladite chambre, pour régler son expansion diamétrale.

8. Anneau selon l'une quelconque des revendications 2 à 7, **caractérisé en ce que** le système de fermeture comporte des moyens pneumatiques (15, 25) pour assurer la fermeture et l'ouverture de l'anneau.

9. Anneau selon la revendication 8, **caractérisé en ce que** les moyens pneumatiques (15, 25) sont portés par le cathéter de réglage (7) et comportent au moins une zone déformable, et **en ce qu'**une ouverture (16) est ménagée dans la paroi (4) de la deuxième partie d'extrémité (6) de la bande, le cathéter de réglage (7) coulissant dans l'ouverture (16) pour former la boucle de l'anneau, la zone déformable (15) étant susceptible d'une part de former localement une excroissance en cas d'augmentation de pression dans le cathéter, ladite excroissance prenant appui contre les parois intérieures de la bande (2) pour bloquer l'anneau en position de fermeture, et d'autre part de reprendre sa forme de repos en cas de retour à la pression normale, pour permettre le coulissement libre du cathéter de réglage (7) et le desserrage de la boucle.

10. Anneau selon la revendication 8, **caractérisé en ce que** la zone déformable (15) est une zone de moindre résistance formée sur le cathéter de réglage (7).

11. Anneau selon la revendication 9 ou 10, **caractérisé en ce que** la deuxième partie d'extrémité (6) de la bande souple (2) est pourvue d'un manchon creux (20), ledit manchon comportant l'ouverture (16) de manière à ce que la première partie d'extrémité (5) de la bande (2) puisse s'insérer dans le manchon (20).

12. Anneau selon la revendication 11, **caractérisé en ce que** la première partie d'extrémité (5) de la bande souple est de forme convergente et comporte la zone déformable qui est de forme triangulaire en coupe transversale.

13. Anneau selon l'une quelconque des revendications 1 à 12, **caractérisé en ce que** la section transversale de la bande (2) est de forme oblongue.

14. Anneau selon l'une des revendications 1 à 13, **caractérisé en ce que** le cathéter de réglage (7) est relié à un dispositif de mise en compression /décompression (8).

## Patentansprüche

1. Magenband aus einem elastischen Band (2) mit einem ersten und zweiten Endstück (5, 6), das dazu bestimmt ist um den Magen geschlossen zu werden in Richtung auf seine zwei Endstücke (5, 6) mittels einem Schließsystem zur Verminderung des Durchmessers der Magenöffnung durch Bilden einer Schleife, **dadurch gekennzeichnet, dass** das Band mindestens ein Griffelement (11, 12, 13) aufweist, das von der Schleife nach außen vorsteht, um das Schließen und das Lösen der zwei Endstücke (5, 6) zu erleichtern.

2. Magenband gemäß Anspruch 1, **dadurch gekennzeichnet, dass** das Band neben dem ersten Endstück (5) mindestens ein erstes Griffelement (11) aufweist, das es ermöglicht, das erste Endstück (5) von dem zweiten Endstück (6) zu lösen.

3. Magenband gemäß Anspruch 2, **dadurch gekennzeichnet, dass** das zweite Endstück mindestens ein zweites Griffelement (12) aufweist, das Schließen der Endstücke und/oder Lösen dieser Endstücke ermöglicht.

4. Magenband gemäß Anspruch 3, **dadurch gekennzeichnet, dass** das zweite Endstück (6) zudem mindestens ein drittes Griffelement (13) aufweist, das das Lösen des zweiten Endstücks (6) vom ersten Endstück (5) ermöglicht.

5. Magenband gemäß Anspruch 4, **dadurch gekennzeichnet, dass** die Griffelemente (11, 12, 13) im wesentlichen eine ebene Form haben und sich erstrecken zwischen einem ersten Ende, das einteilig ist mit dem Band, und einem zweiten Ende, das frei ist und das erfasst werden kann durch Klemmen, um das Endstück des Bands, auf dem es angeordnet ist, positiv zu bewegen.

6. Magenband gemäß Anspruch 5, **dadurch gekennzeichnet, dass** das erste Griffelement (11) in eine Richtung gerichtet ist, die dem zweiten Endstück (6) gegenüber liegt, indem das zweite Griffelement (12) in die gleiche Richtung gerichtet ist, wie das erste Griffelement (11) und indem das dritte Griffelement (13) dem zweiten Griffelement (12) gegenüber liegt.

7. Magenband gemäß einem der vorhergehenden Ansprüche, **dadurch gekennzeichnet, dass** das Band (2) eine ringförmige Druckkammer (3) mit einstellbarem Volumen aufweist, die auf der Höhe des ersten Endstücks (5) verbunden ist mittels einem regelbaren Katheter (7) mit einer Regelvorrichtung für den inneren Druck dieser Kammer, um deren Ausweitung in Richtung des Durchmessers zu regeln.

8. Magenband gemäß einem der Ansprüche 2 bis 7, **dadurch gekennzeichnet, dass** das Schließsystem pneumatische Mittel (15, 25) aufweist, um das Schließen und das Öffnen des Bands zu gewährleisten.

9. Magenband gemäß Anspruch 8, **dadurch gekennzeichnet, dass** die pneumatischen Mittel (15, 25) gehalten sind von dem regelbaren Katheter (7) und mindestens einen verformbaren Bereich aufweisen und dadurch, dass eine Öffnung (16) in der Wandung (4) des zweiten Endstücks (6) des Bands untergebracht ist, wobei das regelbare Katheter (7) in der Öffnung (16) gleitet, um die Schleife des Bands zu bilden, wobei der verformbare Bereich (15) einerseits ausgelegt ist, um örtlich eine Ausformung im Fall von Druckzunahme im Katheter zu bilden, wobei die Ausformung sich gegen die inneren Wandungen des Bands (2) abstützt, um das Magenband in geschlossener Stellung zu blockieren und andererseits seine Ruhestellung wieder einzunehmen im Fall der Wiederkehr von normalem Druck, um das freie Gleiten des regelbaren Katheters (7) zu ermöglichen und das Lösen der Schleife.

10. Magenband gemäß Anspruch 8, **dadurch gekennzeichnet, dass** der verformbare Bereich (15) ein Bereich geringeren Widerstands ist, der auf dem regelbaren Katheter (7) gebildet ist.

11. Magenband gemäß Anspruch 9 oder 10, **dadurch gekennzeichnet, dass** das zweite Endstück (6) des elastischen Bands (2) versehen ist mit einem hohlen Griff (20), wobei der Griff die Öffnung (16) so aufweist, dass das erste Endstück (5) des Bands (2) in den Griff (20) eingeführt werden kann.

12. Magenband gemäß Anspruch 11, **dadurch gekennzeichnet, dass** das erste Endstück (5) des elastischen Bands eine konvergente Form aufweist und den verformbaren Bereich aufweist, der im Querschnitt dreieckige Form hat.

13. Magenband gemäß einem der Ansprüche 1 bis 12, **dadurch gekennzeichnet, dass** der Querschnitt des Bands (2) längliche Form aufweist.

14. Magenband gemäß einem der Ansprüche 1 bis 13, **dadurch gekennzeichnet, dass** das regelbare Katheter (7) verbunden ist mit einer Vorrichtung (8) zum Komprimieren/Dekomprimieren.

## Claims

1. Gastroplasty ring formed by a flexible band (2) which comprises first (5) and second (6) end parts and which is intended to be closed around the stomach towards its two end parts (5, 6) by a closure system in order to reduce the diameter of the opening of the stoma by forming a loop, **characterised in that** the ring comprises at least one gripping lug (11, 12, 13) which projects towards the outside of the loop in order to facilitate the bringing together and separation of the two end parts (5, 6).

2. Ring according to Claim 1, **characterised in that** the ring comprises, in the vicinity of the first end part (5), at least one first gripping lug (11) which makes it possible to separate the first end part (5) from the second end part (6).

3. Ring according to Claim 2, **characterised in that** the second end part comprises at least one second gripping lug (12) which makes it possible to bring together the end parts and/or to separate these end parts.

4. Ring according to Claim 3, **characterised in that** the second end part (6) also comprises at least one third gripping lug (13) which makes it possible to separate the second end part (6) from the first end part (5).

5. Ring according to Claim 4, **characterised in that** the gripping lugs (11, 12, 13) are substantially flat in shape and extend between a first end which is fixed to the ring and a second end which is free and which can be seized by gripping in order to positively move the end part of the ring on which it is mounted.

6. Ring according to Claim 5, **characterised in that** the first gripping lug (11) is directed in the opposite direction to the second end lug (6), **in that** the second gripping lug (12) is oriented in the same direction as the first gripping lug (11), and **in that** the third gripping lug (13) is opposed to the second gripping lug (12).

7. Ring according to one of the preceding claims, **characterised in that** the band (2) comprises an annular compression chamber (3) with adjustable volume which is connected, at the first end part (5), by an adjustment catheter (7) to a device for adjusting the internal pressure of the said chamber, in order to adjust its diametral expansion.

8. Ring according to any one of Claims 2 to 7, **characterised in that** the closure system comprises pneumatic means (15, 25) for providing the closure and opening of the ring.

9. Ring according to Claim 8, **characterised in that** the pneumatic means (15, 25) are carried by the adjustment catheter (7) and comprise at least one deformable area, and **in that** an opening (16) is provided in the wall (4) of the second end part (6) of the band, the adjustment catheter (7) sliding in the opening (16) in order to form the loop of the ring, the deformable area (15) being able on the one hand to form locally a protrusion in the case of increase in pressure in the catheter, the said protrusion bearing against the internal walls of the band (2) in order to block the ring in the closure position, and on the other hand to resume its idle shape in the case of return to normal pressure, to allow the free sliding of the adjustment catheter (7) and the loosening of the loop.

10. Ring according to Claim 8, **characterised in that** the deformable area (15) is an area of lesser resistance formed on the adjustment catheter (7).

11. Ring according to Claim 9 or 10, **characterised in that** the second end part (6) of the flexible band (2) is provided with a hollow sleeve (20), the said sleeve comprising the opening (16) so that the first end part (5) of the band (2) can be inserted into the sleeve (20).

12. Ring according to Claim 11, **characterised in that** the first end part (5) of the flexible band is convergent in shape and comprises the deformable area which is triangular in shape in transverse section.

13. Ring according to any one of Claims 1 to 12, **characterised in that** the transverse section of the band (2) is oblong in shape.

14. Ring according to one of Claims 1 to 13, **characterised in that** the adjustment catheter (7) is connected to a device for putting under compression/decompression (8).
